# EUROPEAN PATENT APPLICATION

(11) **EP 4 779 033 A1**
(43) Date of publication of application: **22.07.2026**
(21) Application number: 24865904.7
(22) Date of filing: 13.09.2024
(51) Int. Cl.: C12Q 1/6876

(54) **COMPOSITION AND DETECTION METHOD FOR DETECTING BETA-CASEIN A1 TYPE GENE AND BETA-CASEIN A2 TYPE GENE IN MILK**

(30) Priority: 15.09.2023 KR 20230123179; 29.12.2023 KR 20230197581
(71) Applicant: Mico Biomed Co., Ltd., Seongnam-si, Gyeonggi-do 13449 (KR)
(72) Inventor: KIM, Hyun Sun, Seongnam-si, Gyeonggi-do 13449 (KR); YOON, Ji Hye, Seongnam-si, Gyeonggi-do 13449 (KR); CHOI, Yun Jeong, Seongnam-si, Gyeonggi-do 13449 (KR)
(74) Representative: Gille Hrabal Partnerschaftsgesellschaft mbB Patentanwälte
(86) International application number: PCT/KR2024/014006
(87) International publication number: WO 2025/058465

(57) **Abstract**

The present application relates to a composition and a detection method which are capable of detecting whether a β-casein A1 type gene and a β-casein A2 type gene are mixed in milk, and have an effect that the β-casein A1 type gene and the β-casein A2 type gene can be accurately detected even when mixed at a low concentration.

## Description

### [Technical Field]

The present application relates to a composition for detecting a β-casein A1 type gene and a β-casein A2 type gene in milk, and a detection method thereof.

### [Background Art]

Milk is a major source of protein in the modern human diet and is a complete food that provides a balanced array of nutrients. Milk typically contains about 30 grams of protein per liter, of which casein is the largest component of protein, accounting for about 80%, and beta-casein accounts for about 37% of casein. β-Casein is further classified into an A1 type, A1/A2 mixed type, and A2 type, and if the 67th amino acid of the 209 amino acids that make up β-casein is histidine, it is named β-casein A1 type, and if it is proline, it is named β-casein A2 type. If the 67th amino acid of β-casein is histidine, phosphorylation is induced to generate a peptide of 7 linked amino acids. The peptide thus generated is named B-casomorphin-7 peptide (BCM-7). BCM-7 is known to be involved in indigestion, inflammation, allergies, and the like. On the other hand, when the 67th amino acid is proline, that is, the A2 type β-casein, phosphorylation is not induced and thus BCM-7 is not produced in milk.

Korean Patent No. 10-2297680 is a patent regarding a method for selecting an individual capable of producing high-quality milk with a high content of β-casein A2 type and an appropriate number of somatic cells and urea nitrogen content. However, after analyzing the genetic traits of dairy cows capable of producing β-casein A2 type milk and selecting individuals, several processes are required to produce milk. In other words, several stages of processes such as separate breeding of dairy cows, separate production, separate milk collection, and separate processing are required depending on the traits. However, there is a possibility of incorporation of β-casein A1 type milk during this process, and to date, methods for confirming the incorporation of β-casein A1 type milk have been unclear or extremely time-consuming, making it difficult to practically apply the method. In addition, there are limitations in the quality control of β-casein A2 type milk, as there are no established technical methods for examining commercially available milk.

Therefore, there is a need for developing a method capable of detecting whether the β-casein A1 type and β-casein A2 type are incorporated into milk.

### [Disclosure]

### [Technical Problem]

The problem to be solved by the present application is to provide a composition capable of detecting whether a β-casein A1 type gene and a β-casein A2 type gene are incorporated into milk.

Another problem to be solved by the present application is to provide a kit capable of detecting whether a β-casein A1 type gene and a β-casein A2 type gene are incorporated into milk.

Still another problem to be solved by the present application is to provide a method capable of detecting whether a β-casein A1 type gene and a β-casein A2 type gene are incorporated into milk.

### [Technical Solution]

One embodiment of the present application provides a composition for detecting a β-casein A1 type gene and a β-casein A2 type gene in milk, including a forward primer consisting of a base sequence of SEQ ID NO: 1; a reverse primer consisting of a base sequence of SEQ ID NO: 2; a probe consisting of a base sequence of SEQ ID NO: 3; and a probe consisting of a base sequence of SEQ ID NO: 4.

In one embodiment of the present application, the composition further includes a probe including a base sequence of SEQ ID NO: 5 and is capable of determining a low concentration of the β-casein A1 type gene.

In one embodiment of the present application, the detection may be performed by a real-time polymerase chain reaction (real-time PCR).

One embodiment of the present application provides a diagnostic kit for detection including the composition.

One embodiment of the present application provides a method for detecting whether a β-casein A1 type gene and a β-casein A2 type gene are incorporated into milk, the method including: performing a real-time polymerase chain reaction (real-time PCR) using a composition including a forward primer consisting of a base sequence of SEQ ID NO: 1, a reverse primer consisting of a base sequence of SEQ ID NO: 2, a probe consisting of a base sequence of SEQ ID NO: 3, and a probe consisting of a base sequence of SEQ ID NO: 4.

In one embodiment of the present application, the performing of the real-time polymerase chain reaction may include initially denaturing the DNA of milk at 90°C to 97°C; and repeatedly performing adding the composition to denature the DNA of milk at 90°C to 97°C and annealing at 50°C to 60°C.

In one embodiment of the present application, the performing of the real-time polymerase chain reaction may include, after the initially denaturing of the DNA of milk, binding a probe consisting of a base sequence of SEQ ID NO: 5 to the β-casein A2 type gene at 65°C to 75°C to suppress amplification; and may detect a low concentration of the β-casein A1 type gene.

### [Advantageous Effects]

The composition and diagnostic kit of the present application can detect whether a β-casein A1 type gene and a β-casein A2 type gene are incorporated into milk, and have an advantage of being able to detect whether the β-casein A1 type gene is incorporated even when incorporated at a low concentration.

The composition and diagnostic kit of the present application further include a β-casein A2 type gene amplification suppression probe, and thus have an advantage of being able to detect whether the β-casein A1 type gene is incorporated even when incorporated at a low concentration.

### [Description of Drawings]

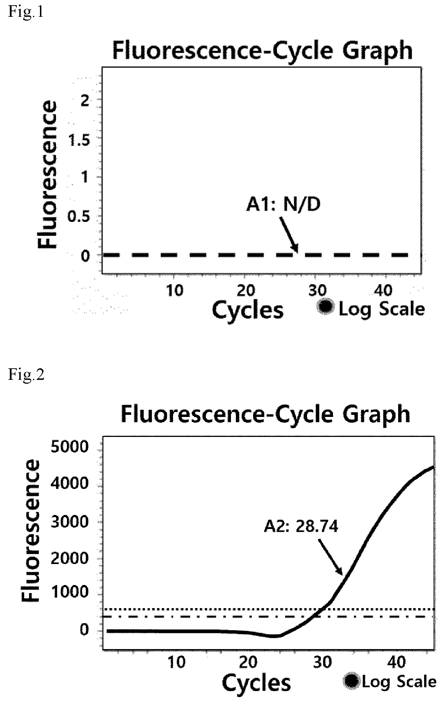

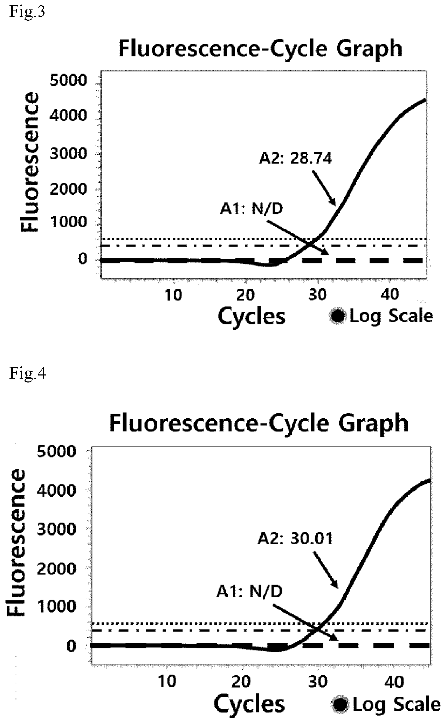

FIG. 1 illustrates the results of a β-casein A1 type gene detection reagent in milk into which the β-casein A1 type milk of Example 1 is not incorporated, FIG. 2 illustrates the results of a β-casein A2 type gene detection reagent, and FIG. 3 illustrates the results of the β-casein A1 type gene and β-casein A2 type gene detection reagents together.
FIG. 4 illustrates the results of the β-casein A1 type gene detection reagent and the β-casein A2 type gene detection reagent in milk into which 1 wt% of the β-casein A1 type milk of Example 2 is incorporated.

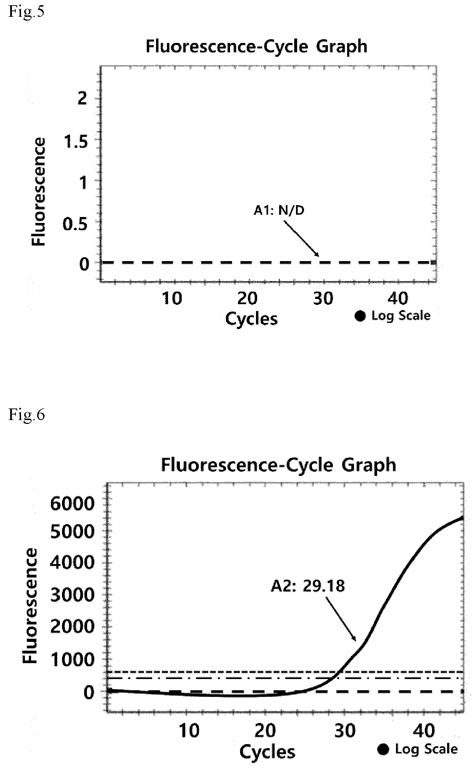

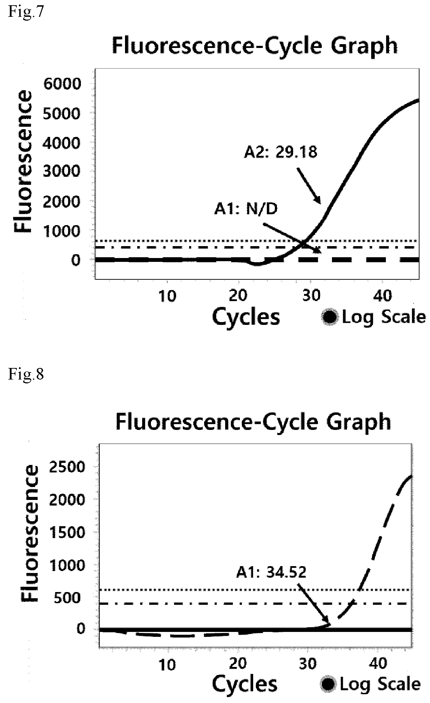

FIG. 5 illustrates the results of the β-casein A1 type gene detection reagent in milk into which the β-casein A1 type milk of Example 3 is not incorporated, FIG. 6 illustrates the results of the β-casein A2 type gene detection reagent, and FIG. 7 illustrates the results of the β-casein A1 type gene and the β-casein A2 type gene detection reagents together.

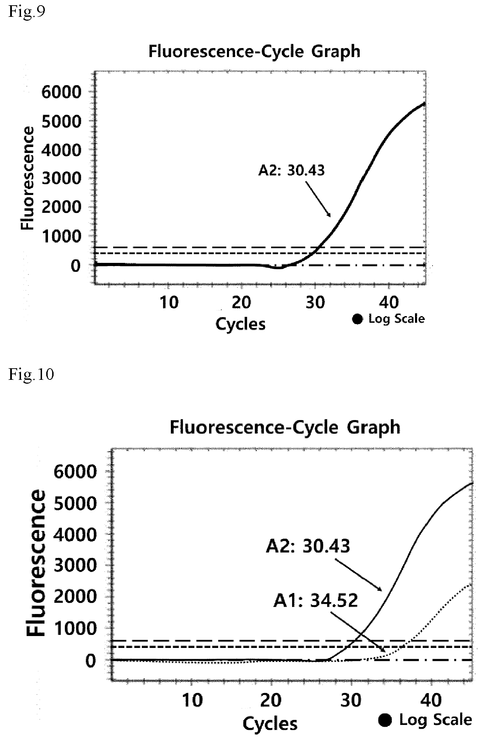

FIG. 8 illustrates the results of the β-casein A1 type gene detection reagent in milk into which 1 wt% of the β-casein A1 type milk of Example 4 is incorporated, FIG. 9 illustrates the results of a β-casein A2 type gene detection reagent, and FIG. 10 illustrates the results of the β-casein A1 type gene and β-casein A2 type gene detection reagents together.

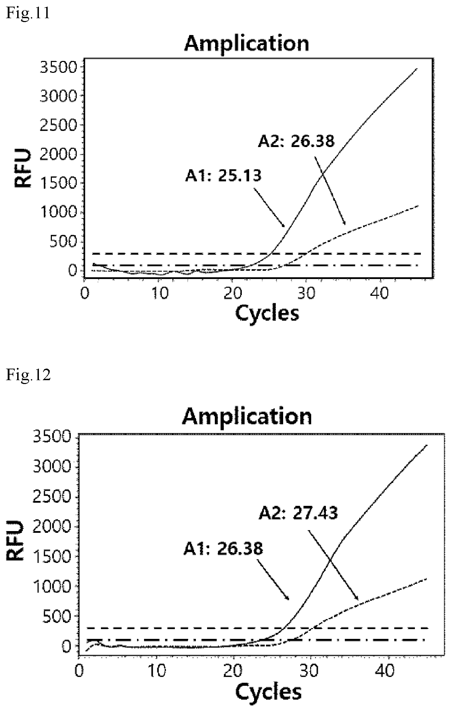

FIG. 11 illustrates the results of the β-casein A1 type gene detection reagent and the results of the β-casein A2 type gene detection reagent in milk into which the β-casein A1 type milk of Comparative Example 1 is not incorporated, and FIG. 12 illustrates the results of the β-casein A1 type gene detection reagent and the results of the β-casein A2 type gene detection reagent in milk into which 1 wt% of the β-casein A1 type milk is incorporated.

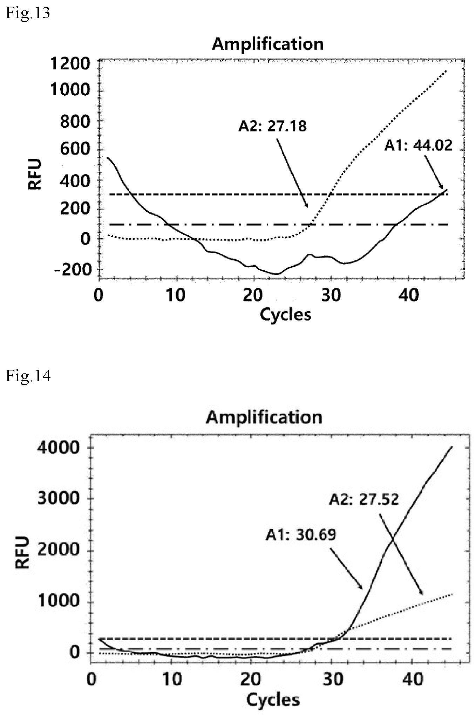

FIG. 13 illustrates the results of the β-casein A1 type gene detection reagent and the results of the β-casein A2 type gene detection reagent in milk into which the β-casein A1 type milk of Comparative Example 2 is not incorporated, and FIG. 14 illustrates the results of the β-casein A1 type gene detection reagent and the results of the β-casein A2 type gene detection reagent in milk into which 1 wt% of the β-casein A1 type milk is incorporated.

### [Modes of the Invention]

Hereinafter, the present application will be described in more detail.

The following specific functional descriptions are merely illustrative examples for describing embodiments according to the concept of the present application, and the embodiments according to the concept of the present application may be embodied in various forms and should not be construed as being limited to the embodiments described in the present specification.

Since the embodiments according to the concept of the present application may be variously modified and take various forms, specific embodiments will be described in detail in the present specification. However, this is not intended to limit the embodiments according to the concept of the present application to specific disclosed forms, and it is to be understood that all the changes, equivalents and substitutions included in the spirit and scope of the present application are included in the present invention.

The terms used in the present specification are used only to describe specific embodiments, and are not intended to limit the present application. Singular expressions include plural expressions unless the context clearly indicates otherwise.

Unless defined otherwise, all terms used herein, including technical or scientific terms, have the same meaning as commonly understood by a person with ordinary skill in the art to which the present application pertains. Terms such as those defined in commonly used dictionaries should be interpreted as having a meaning consistent with the meaning in the context of the relevant art and should not be interpreted in an ideal or overly formal sense unless explicitly defined in the present specification.

One embodiment of the present application provides a composition capable of detecting a β-casein A1 type gene and a β-casein A2 type gene in milk to determine whether they are incorporated, including a forward primer consisting of a base sequence of SEQ ID NO: 1; a reverse primer consisting of a base sequence of SEQ ID NO: 2; a probe consisting of a base sequence of SEQ ID NO: 3; and a probe consisting of a base sequence of SEQ ID NO: 4.

In the present application, "determination" includes quantitative and/or qualitative analysis, including the detection of presence or absence and the measurement of concentration, and these methods are known in the art, and a person skilled in the art will be able to select an appropriate method for the implementation of the present application. In the present application, "determination" means deriving a result regarding the presence or absence of a target gene in the milk being tested.

In the present application, "casein" refers to a type of phosphoprotein as the main protein in milk, and is composed of α-, β-, and κ-casein and other trace amounts of casein. Since the average content of nitrogen contained in casein is 15.67%, when quantifying casein (or milk protein), the amount is calculated by multiplying the nitrogen amount by a coefficient of 6.38. Casein has a property of precipitating at pH 4.6 because the average isoelectric point of casein is pH 4.6. When milk is acidified, the calcium and some phosphorus in a casein complex become soluble and are released into whey, and casein is precipitated without containing salt.

The aforementioned β-casein is classified into a β-casein A1 type and β-casein A2 type, and the β-casein A1 type differs from the β-casein A2 type by a single amino acid. In distinguishing the traits of the β-casein A1 type and β-casein A2 type of dairy cows, it is possible to determine whether the 200th base of the *CSN2* gene is A or C. Specifically, if the gene sequence is CAT, it can be determined as the β-casein A1 type which expresses the 67th amino acid as histidine, and if the gene sequence is CTT, it can be determined as the β-casein A2 type which expresses the 67th amino acid as proline. This single amino acid difference is very important for the enzymatic digestion of β-caseins in the intestine, and the presence of histidine at position 67 allows a protein fragment including seven amino acids named B-casomorphin-7 (BCM-7) to be generated during enzymatic digestion. Therefore, BCM-7 is a digestion product of β-casein A1 and is responsible for indigestion, inflammation, allergies, and the like. In the case of the β-casein A2 type, since position 67 is occupied by proline, which prevents cleavage of amino acid bonds, BCM-7 is not produced as a digestion product.

In order to determine whether the 200th base of the *CSN2* gene is A or C, a single base sequence, SNP (rs43703011), needs to be distinguished, and therefore a specific reaction needs to be induced. When using an existing DNA probe, it was difficult to distinguish because dTm per 1 mer of base was low. The probe according to the present application has an advantage in that the ability to distinguish a single base sequence is improved using PNA instead of DNA.

For individual analysis of dairy cows, it is possible to select the genetic traits of dairy cows because the β-casein A1 trait and the β-casein A2 trait are present at either 100% or 50%. However, milk with the β-casein A1 trait may be incorporated during the milk production process, and in this case, it is necessary to distinguish between the β-casein A1 trait and the β-casein A2 trait in milk, rather than selecting the genetic traits of an individual. Furthermore, when β-casein A1 type milk is incorporated at a low concentration, the fluorescence value increases as the amount of non-specific binding increases due to the characteristics of a nucleic acid amplification method using a dual-labeled probe, so there is a problem in that accuracy deteriorates when simply analyzing the Ct value range, as in Korean Patent No. 10-2297680.

The present application uses a PNA probe that suppresses the amplification of the β-casein A2 type gene, and thus has an advantage in that the accuracy of detection of the β-casein A1 type gene is increased by suppressing the amplification of the β-casein A2 type gene.

The primer of the present invention may incorporate additional features that do not change the basic properties. That is, nucleic acid sequences may be modified using many means known in the art. Examples of such modifications include methylation, capping, substitution with one or more homologues of a nucleotide, and modification of nucleotides with uncharged linkers such as phosphonates, phosphotriesters, phosphoramidates or carbamates, or with charged linkers such as phosphorothioates or phosphorodithioates. Further, nucleic acids may have one or more additional covalently bonded moieties, such as nucleases, toxins, antibodies, signal peptides, proteins such as poly-L-lysine, intercalators such as acridine or psoralen, chelating agents such as metals, radioactive metals, and iron oxide metals, and alkylating agents. In addition, the primer of the present application may include a nucleotide analogue, for example, a phosphorothioate, an alkyl phosphorothioate or a peptide nucleic acid, or may include an intercalating agent.

In the present application, the term "probe" refers to a nucleic acid fragment corresponding to several to several hundred bases that can specifically bind to DNA or RNA, and may be prepared in the form of an oligonucleotide probe, a single stranded DNA probe, a double stranded DNA probe, an RNA probe, a peptide nucleic acid (PNA) probe, and the like, but a PNA probe may be preferably used. The peptide nucleic acid (PNA) has excellent gene recognition ability due to its structural characteristics, and has an advantage in that a more stable bond can be maintained because the binding force of PNA-DNA is stronger than bonds between DNAs.

The probe may be chemically synthesized using a phosphoramidite solid support method or other well-known methods. These probes may be modified using many means known in the art, as long as they are effective in detecting the β-casein A1 type and β-casein A2 type. Examples of such modifications include methylation, capping, substitution with one or more homologues of a natural nucleotide, and modification between nucleotides, such as modification with uncharged linkers (for example, methylphosphonate, phosphotriester, phosphoroamidate, carbamate, and the like) or charged linkers (for example, phosphorothioate, phosphorodithioate, and the like). The nucleic acid may contain one or more additional covalently linked moieties, such as proteins (for example, nucleases, toxins, antibodies, signal peptides, poly-L-lysine, and the like), intercalators (for example, acridine, psoralen, and the like), chelating agents (for example, metals, radioactive metals, iron, oxidizing metals, and the like), and alkylating agents.

Furthermore, the probe sequence of the present application may be modified using a label capable of providing a detectable signal, either directly or indirectly. The probe may include a label capable of being detected using spectroscopic, photochemical, biochemical, immunochemical, or chemical means. Useful labels may include 32P, fluorescent dyes, electron-dense reagents, enzymes (typically those used in ELISA), biotins, haptens, and proteins for which antisera or monoclonal antibodies are available.

In one embodiment of the present application, the probe may be labeled with a fluorescent dye that acts as a reporter. The probe also has a second dye that acts as a quencher, and includes a fluorescent substance and a quencher at the 5' end and the 3' end, respectively, and when a plurality of probes are included, the fluorescent substances included at the 5' end of each probe may be all the same. This reporter dye is measured at a predetermined wavelength, thereby enabling the detection and identification of an amplified target. A probe fluorescence signal in a complete state is suppressed by a quencher dye.

In addition, one or more additional probes, for example an internal reference control or another target probe or another nucleic acid, may be labeled with a unique reporter fluorescent dye different from a fluorescent dye label associated with the probe. In this case, by measuring a specific reporter dye at a predetermined wavelength, the amplified target and the one or more additional probes can be simultaneously detected and identified.

The fluorescent substance may be any one or more selected from the group consisting of 6-carboxyfluorescein (6-FAM), 5-carboxyfluorescein (5-FAM), hexachloro-6-carboxyfluorescein, tetrachloro-6-carboxyfluorescein, 2',4',5',7'-tetrachloro-6-carboxy-4,7-dichlorofluorescein (HEX), fluorescein chlorotriazinyl, rhodamine green, rhodamine red, tetramethylrhodamine, fluorescein isothiocyanate (FITC), Oregon green, Alexa fluor, 6-carboxy-4',5'-dichloro-2',7'-dimethoxyfluorescein (JOE), 6-carboxyl-Xrhodamine (ROX), tetrachloro-fluorescein (TET), tetramethylrhodamine isothiocyanate (TRITC), 6-carboxytetramethyl-rhodamine (TAMRA), N-(1-naphthyl) ethylenediamine (NED), Texas red, cyanine-3 (Cy3), and cyanine-5 (Cy5).

The quencher may be any one or more selected from the group consisting of black hole quencher 1 (BHQ1), black hole quencher 2 (BHQ2), black hole quencher 3 (BHQ3), 6-carboxytetramethyl-rhodamine (TAMRA), a nonfluorescent quencher (NFQ), Dabcyl, Eclipse, Deep Dark Quencher (DDQ), Blackberry Quencher, and Iowa black.

In the present application, the term "real-time polymerase chain reaction (Real-time PCR)" refers to a method of amplifying DNA in real time and simultaneously measuring the amplified amount based on the polymerase chain reaction.

Since the real-time polymerase chain reaction can monitor and confirm the increase in PCR amplification products in real time, an additional electrophoresis analysis method does not need to be used, and compared to the existing PCR method that confirms PCR amplification products at the end point, it can interpret PCR quickly and easily and has a low risk of contamination. Furthermore, the method for detecting real-time polymerase chain reaction amplification products uses a probe that is specific for a target sequence and has a fluorescent dye bonded to it, and has an advantage in that even similar sequences can be distinguished and detected due to its high detection specificity.

In one embodiment of the present application, the composition may include a PCR premix; a primer and probe set; and an internal control (IC).

In the present application, the term "PCR premix" means a reaction mixture prepared in advance before a PCR reaction, which is a ready-to-use reagent that allows an immediate PCR reaction by adding a DNA sample to be analyzed.

The PCR premix may use a master mix further including a mixture of dNTPs (dATP, dCTP, dGTP, dTTP, dUTP or variants with similar functions), magnesium chloride (MgCl₂), DNA polymerase or buffer solution.

One embodiment of the present application provides a diagnostic kit capable of determining the presence or absence of incorporation by detecting the β-casein A1 type gene and the β-casein A2 type gene of the milk.

In the present application, the term "kit" refers to a set of test specimens for selecting a β-casein A1 type gene and a β-casein A2 type gene in milk, can confirm the presence or absence of a specific SNP in milk, and preferably includes primers and probes for the target SNP of the present application, but is not limited thereto.

The diagnostic kit is not limited to a specific form, and may be implemented in various forms. In one embodiment, the diagnostic kit may include lyophilized primers contained in a container such as a disposable PCR tube. For example, a disposable PCR tube containing a composition using a primer and probe set of SEQ ID NOS: 1 to 3 in the form of a lyophilized powder may be included in the kit.

The diagnostic kit may contain a reaction mixture including the primers and probes, polymerase, and buffer, and the like in the form of a liquid in a container such as a PCR tube. This may be stored frozen and thawed for use, when necessary. The diagnostic kit has an advantage in that the convenience and properties of detection are increased because no separate process is required other than adding a sample.

One embodiment of the present application provides a method for detecting whether a β-casein A1 type gene and a β-casein A2 type gene are incorporated into milk, the method including: performing a real-time polymerase chain reaction (real-time PCR) method using a composition including a forward primer consisting of a base sequence of SEQ ID NO: 1; a reverse primer consisting of a base sequence of SEQ ID NO: 2; a probe consisting of a base sequence of SEQ ID NO: 3; and a probe consisting of a base sequence of SEQ ID NO: 4.

In the detection method, the performing of the real-time polymerase chain reaction may include initially denaturing the DNA of the milk at 90°C to 97°C, more specifically at 93°C to 96°C. The initial denaturing step may be performed for 5 seconds to 10 minutes, more specifically for 10 seconds to 5 minutes to dissociate the double strands of the DNA.

In addition, the performing of the real-time polymerase chain reaction may include, after the initial denaturing step, suppressing amplification by binding a probe consisting of a base sequence of SEQ ID NO: 5 to the β-casein A2 type gene at 65°C to 75°C, more specifically at 68°C to 72°C. The suppressing of the amplification is performed for 30 seconds to 15 minutes, more specifically for 3 minutes to 10 minutes to suppress the amplification of the β-casein A2 type gene, thereby having an advantage of being able to solve a problem in that accuracy deteriorates due to an increase in fluorescence value when using a dual-labeled probe and to enable accurate detection even when the β-casein A1 type gene is included at a low concentration. In this case, there is an advantage in that detection is possible even when the proportion of the β-casein A1 type gene among the β-casein A1 type gene and the β-casein A2 type gene is incorporated at a low concentration of 0.1 or less, 0.01 or less, or 0.001 or less.

After the suppressing of the amplification, the method may include adding the composition, denaturing at 90°C to 97°C, more specifically at 93°C to 96°C, and repeatedly performing annealing at 50°C to 60°C, more specifically at 54°C to 58°C to amplify the gene.

In the amplifying of the gene, denaturation may be performed for 1 second to 15 seconds, specifically for 3 seconds to 10 seconds, and annealing may be performed for 3 seconds to 30 seconds, specifically for 5 seconds to 20 seconds. In the performing of the annealing, when the temperature is higher than 60°C, the primers may not be annealed, so that a PCR product may not be generated, and when the temperature is lower than 50°C, non-specific annealing may occur, so that an accurate PCR product may not be generated.

In the amplifying of the gene, denaturation may be performed for 1 second to 15 seconds, specifically for 3 seconds to 10 seconds, and annealing may be performed for 3 seconds to 30 seconds, specifically for 5 seconds to 20 seconds. In the performing of the annealing, when the temperature is higher than 60°C, the primers may not be annealed, so that a PCR product may not be generated, and when the temperature is lower than 50°C, non-specific annealing may occur, so that an accurate PCR product may not be generated.

Furthermore, the milk may be used diluted or undiluted before being added dropwise onto the sample pad of the diagnostic kit of the present application.

Hereinafter, the present application will be described in more detail through Preparation Examples, Examples, Comparative Examples, and Experimental Examples. These examples are only for exemplifying the present application, and it will be obvious to those of ordinary skill in the art that the scope of the present application should not be construed as being limited by these examples.

### 1. Preparation of primers and probes for SNP detection in CNS2

Primers and probes capable of detecting a β-casein A1 type gene and a β-casein A2 type gene in milk were prepared, each sequence is as shown in the following Table 1, and the combinations used for each example are as shown in Table 2.

**[Table 1]**

| SEQ ID NO: | Target | Description | Base sequence (5'-3') | Label fluorescence |
|---|---|---|---|---|
| 1 | A1 and A2 | Forward primer | GCCCAGACACAGTCTCT AGTC | |
| 2 | A1 and A2 | Reverse primer | CACCACAGGGGTTTGAG TAAGAGG | |
| 3 | A1 | PNA probe | CCATCCATAACAGCC | Cy5 |
| 4 | A2 | PNA probe | CCATCCCTAACAGC | ROX |
| 5 | A2 | Amplification suppression PNA probe | GCCCATCCCTAACAGCC | |

**[Table 2]**

| Classification | Target | Combination | |
|---|---|---|---|
| Examples 1 and 2 | A1 | SEQ ID NOS: 1, 2, and 3 | |
| | A2 | SEQ ID NOS: 1, 2, and 4 | |
| Examples 3 and 4 | A1 | SEQ ID NOS: 1, 2, 3, and 5 | |
| | A2 | SEQ ID NOS: 1, 2, 4, and 5 | |

### 2. Amplification of genes by real-time polymerase chain reaction

### <Example 1>

Milk Sample 1 into which β-casein A1 type milk had not been incorporated was prepared, nucleic acids were extracted, and then results were analyzed by amplifying the nucleic acids using a mixed solution of primers and probes of SEQ ID NOS: 1 to 3 and a mixed solution of primers and probes of SEQ ID NOS: 1, 2, and 4, and the operating conditions of a real-time polymerase chain reaction device (product name: PCR316, manufacturer: MiCo BioMed) are as shown in the following Table 3.

**[Table 3]**

| Step | Temperature (°C) | Time | Cycle |
|---|---|---|---|
| Initial denaturation | 95 | 3 minutes | 1 |
| A2 gene binding | 70 | 5 minutes | 1 |
| Denaturation | 95 | 5 seconds | 45 |
| Annealing | 56 | 10 seconds | |

In Table 3, the nucleic acids of the β-casein A1 type and β-casein A2 type genes were amplified and detected by dissociating the DNA double strand during the initial denaturing step, and then repeating the denaturing and annealing steps.

The experiment was performed twice on Milk Sample 1, the results are shown in the following Table 4, and the second results are illustrated in FIGS. 1 to 3. The results of a β-casein A1 type gene detection reagent are illustrated in FIG. 1, the results of a β-casein A2 type gene detection reagent are illustrated in FIG. 2, and the results of the β-casein A1 type and β-casein A2 type detection reagents are illustrated together in FIG. 3. It could be confirmed that the β-casein A2 type was detected accurately and specifically.

**[Table 4]**

| Example 1 | Cy5(A1) | ROX(A2) |
|---|---|---|
| | A1: 0 wt%, A2: 100 wt% | A1: 0 wt%, A2: 100 wt% |
| A1 mix | N/D | |
| | N/D | |
| A2 mix | | 28.75 |
| | | 28.74 |

### <Example 2>

Milk Sample 2 into which 1 wt% of β-casein A1 type milk and 99 wt% of β-casein A2 type milk had been incorporated was prepared, and the same process as in Example 1 was performed. The experiment was performed twice, the results are shown in the following Table 5, and the β-casein A1 type gene detection reagent results and β-casein A2 type gene detection reagent results of the second result are illustrated in FIG. 4. The β-casein A2 type was accurately detected, but the β-casein A1 type mixed at a low concentration (1 wt%) was not detected.

**[Table 5]**

| Example 2 | Cy5(A1) | ROX(A2) |
|---|---|---|
| | A1: 1 wt%, A2: 99 wt% | A1: 1 wt%, A2: 99 wt% |
| A1 mix | N/D | |
| | N/D | |
| A2 mix | | 30.22 |
| | | 30.01 |

### <Example 3>

Milk Sample 1 was prepared in the same manner as in Example 1, nucleic acids were extracted, and then results were analyzed by amplifying the nucleic acids using a mixed solution of primers and probes of SEQ ID NOS: 1 to 3, and 5 and a mixed solution of primers and probes of SEQ ID NOS: 1, 2, and 4, and the operating conditions of a real-time polymerase chain reaction device are as shown in Table 2.

In Table 2, in the initial denaturing step, the DNA double strand is dissociated, and in the β-casein A2 type gene binding step, the PNA probe of SEQ ID NO: 5 binds to the β-casein A2 type gene to suppress amplification. Thereafter, the denaturing step and the annealing step were repeated to amplify and detect the nucleic acids of the β-casein A1 type gene and the β-casein A2 type gene.

The experiment was performed twice on Milk Sample 1, the results are shown in the following Table 6, and the second results are illustrated in FIGS. 5 to 7. The results of a β-casein A1 type gene detection reagent are illustrated in FIG. 5, the results of a β-casein A2 type gene detection reagent are illustrated in FIG. 6, and the results of the β-casein A1 type and β-casein A2 type detection reagents are shown together in FIG. 7. It could be confirmed that the β-casein A2 type was detected accurately and specifically.

**[Table 6]**

| Example 3 | Cy5(A1) | ROX(A2) |
|---|---|---|
| | A1: 0 wt%, A2: 100 wt% | A1: 0 wt%, A2: 100 wt% |
| A1 mix | N/D | |
| | N/D | |
| A2 mix | | 28.97 |
| | | 29.18 |

### <Example 4>

Milk Sample 2 in which 1 wt% of β-casein A1 type milk and 99 wt% of β-casein A2 type milk had been mixed was prepared, and the same process as in Example 3 was performed. The β-casein A1 type and β-casein A2 type were accurately and specifically detected, and it was confirmed that accurate detection and analysis were possible even when low concentrations of β-casein A1 type milk were mixed. The experiment was performed twice, the results are shown in the following Table 7, and the second results are illustrated in FIGS. 8 to 10. FIG. 8 illustrates the results of a β-casein A1 type gene detection reagent, FIG. 9 illustrates the results of a β-casein A2 type gene detection reagent, and FIG. 10 illustrates the results of the β-casein A1 type gene detection reagent and the results of the β-casein A2 type gene detection reagent.

**[Table 7]**

| Example 4 | Cy5(A1) | ROX(A2) |
|---|---|---|
| | A1: 1 wt%, A2: 99 wt% | A1: 1 wt%, A2: 99 wt% |
| A1 mix | 33.96 | |
| | 34.52 | |
| A2 mix | | 30.07 |
| | | 30.43 |

### <Comparative Example 1>

Milk Sample 1 and Milk Sample 2 in which 1 wt% of β-casein A1 type milk and 99 wt% of β-casein A2 type milk had been mixed were prepared, nucleic acids were extracted, and then the same process as in Example 1 was performed using a mixed solution of primers and probes of SEQ ID NOS: 1, 2, and 6 of the following Table 8 and a mixed solution of primers and probes of SEQ ID NOS: 1, 2, and 7.

**[Table 8]**

| SEQ ID NO: | Target | Description | Base sequence (5'-3') | Label fluorescen ce |
|---|---|---|---|---|
| 1 | A1 and A2 | Forward primer | GCCCAGACACAGTCTC TAGTC | |
| 2 | A1 and A2 | Reverse primer | CACCACAGGGGTTTGA | |
| | | | GTAAGAGG | |
| 6 | A1 | DNA probe | AGAGGCGICCCATCCAT AACAGCCTCGCCTCT | FAM |
| 7 | A2 | DNA probe | GGAGCCGICCCATCCCT AACAGCCTCGGCTCC | TexasRed |

| | | | | |
|---|---|---|---|---|
| *N is a base sequence which may be modified. | | | | |

The experiment was performed twice, the results are shown in the following Table 9, and the β-casein A1 type gene detection reagent results and β-casein A2 type gene detection reagent results of the second result are illustrated in FIGS. 11 and 12. Non-specific detection was observed using a DNA probe as a detection probe for the β-casein A1 type and β-casein A2 type, and it was impossible to determine whether the β-casein A1 type was mixed.

**[Table 9]**

| Comparative Example 1 | FAM(A1) | TEX(A2) | FAM(A1) | TEX(A2) |
|---|---|---|---|---|
| | A1: 0 wt%, A2: 100 wt% | A1: 0 wt%, A2: 100 wt% | A1: 1 wt%, A2: 99 wt% | A1: 1 wt%, A2: 99 wt% |
| A1 mix | 25.17 | | 26.15 | |
| | 25.13 | | 26.38 | |
| A2 mix | | 27.33 | | 29.12 |
| | | 27.22 | | 27.43 |

### <Comparative Example 2>

Milk Sample 1 and Milk Sample 2 in which 1 wt% of β-casein A1 type milk and 99 wt% of β-casein A2 type milk had been mixed were prepared, nucleic acids were extracted, and then the same process as in Comparative Example 1 was performed using a mixed solution of primers and probes of SEQ ID NOS: 1, 2, and 6 of the following Table 10 and a mixed solution of primers and probes of SEQ ID NOS: 1, 2, 5, and 7.

**[Table 10]**

| SEQ ID NO: | Target | Description | Base sequence (5'-3') | Labeling fluorescence |
|---|---|---|---|---|
| 1 | A1 and A2 | Forward primer | GCCCAGACACAGTCTC TAGTC | |
| 2 | A1 and A2 | Reverse primer | CACCACAGGGGTTTGA GTAAGAGG | |
| 6 | A1 | DNA probe | AGAGGCGICCCATCCAT AACAGCCTCGCCTCT | FAM |
| 7 | A2 | DNA probe | GGAGCCGICCCATCCCT AACAGCCTCGGCTCC | TexasRed |
| 5 | A2 | Amplification suppression PNA probe | GCCCATCCCTAACAGCC | |

| | | | | |
|---|---|---|---|---|
| *N is a base sequence which may be modified. | | | | |

The experiment was performed twice, the results are shown in the following Table 11, and the β-casein A1 type gene detection reagent results and β-casein A2 type gene detection reagent results of the second result are illustrated in FIGS. 13 and 14. Even though an amplification suppression PNA probe was used, non-specific detection was observed using a DNA probe as a detection probe for the β-casein A1 type and β-casein A2 type, and it was impossible to determine whether the β-casein A1 type was mixed.

**[Table 11]**

| Comparative Example 2 | FAM(A1) | TEX(A2) | FAM(A1) | TEX(A2) |
|---|---|---|---|---|
| | A1: 0 wt%, A2: 100 wt% | A1: 0 wt%, A2: 100 wt% | A1: 1 wt%, A2: 99 wt% | A1: 1 wt%, A2: 99 wt% |
| A1 mix | 42.69 | | 31.24 | |
| | 44.02 | | 30.69 | |
| A2 mix | | 27.20 | | 29.00 |
| | | 27.18 | | 27.52 |

Although specific parts of the present invention have been described in detail, it is obvious to those skilled in the art that such specific descriptions are only preferred embodiments and the scope of the present invention is not limited thereby. Accordingly, the substantial scope of the present invention will be defined by the appended claims and equivalents thereof.

## Claims

1. A composition for detecting a β-casein A1 type gene and a β-casein A2 type gene in milk, comprising: a forward primer consisting of a base sequence of SEQ ID NO: 1;
a reverse primer consisting of a base sequence of SEQ ID NO: 2;
a probe consisting of a base sequence of SEQ ID NO: 3; and
a probe consisting of a base sequence of SEQ ID NO: 4.

2. The composition of claim 1, wherein the detection is performed by a real-time polymerase chain reaction (real-time PCR).

3. The composition of claim 1, wherein the detection is performed by a real-time polymerase chain reaction (real-time PCR).

4. A diagnostic kit for detecting a β-casein A1 type gene and a β-casein A2 type gene in milk, comprising the composition of any one of claims 1 to 3.

5. A method for detecting whether a β-casein A1 type gene and a β-casein A2 type gene are mixed in milk, the method comprising: performing a real-time polymerase chain reaction (real-time PCR) method using a composition comprising a forward primer consisting of a base sequence of SEQ ID NO: 1,
a reverse primer consisting of a base sequence of SEQ ID NO: 2,
a probe consisting of a base sequence of SEQ ID NO: 3, and
a probe consisting of a base sequence of SEQ ID NO: 4.

6. The method of claim 5, wherein the performing of the real-time polymerase chain reaction comprises initially denaturing the DNA of milk at 90°C to 97°C; and
repeatedly performing adding the composition to denature the DNA of milk at 90°C to 97°C and annealing at 50°C to 60°C.

7. The method of claim 6, wherein the performing of the real-time polymerase chain reaction comprises, after the initially denaturing of the DNA of milk, binding a probe consisting of a base sequence of SEQ ID NO: 5 to the β-casein A2 type gene at 65°C to 75°C to suppress amplification; and detects a low concentration of the β-casein A1 type gene.
